# EUROPEAN PATENT SPECIFICATION

(11) **EP 1 664 767 B1**
(45) Date of publication and mention of the grant of the patent: **14.11.2007**
(21) Application number: 04774922.1
(22) Date of filing: 03.09.2004
(51) Int. Cl.: G01N 33/50

(54) **METHOD FOR DETERMINING THE IMPACT OF A MULTICOMPONENT NATURAL PRODUCT MIXTURE ON THE BIOLOGICAL PROFILE OF A DISEASE WITHIN A GROUP OF LIVING SYSTEMS AND THE DEVELOPMENT AND QUALITY CONTROL OF NATURAL PRODUCT BASED MEDICINE**
VERFAHREN ZUR BESTIMMUNG DER AUSWIRKUNG EINES AUS MEHREREN KOMPONENTEN BESTEHENDEN NATURPRODUKTGEMISCHS AUF DAS BIOLOGISCHE PROFIL EINER KRANKHEIT IN EINER GRUPPE LEBENDER SYSTEME SOWIE ENTWICKLUNG UND QUALITÄTSKONTROLLE VON MEDIZIN AUF NATURPRODUKTBASIS
METHODE PERMETTANT DE DETERMINER L'IMPACT D'UN MELANGE DE PRODUITS NATURELS MULTICOMPOSANTS SUR LE PROFIL BIOLOGIQUE D'UNE MALADIE DANS UN GROUPE DE SYSTEMES VIVANTS

(30) Priority: 05.09.2003 EP 03077804
(43) Date of publication of application: 07.06.2006
(73) Proprietor: Nederlandse Organisatie voor Toegepast-Natuurwetenschappelijk Onderzoek TNO, 2628 VK Delft (NL)
(72) Inventor: WANG, Mei, NL-2341 BV Oegstgeest (NL); WITKAMP, Renger, NL-3662 LM Wijk bij Duurstede (NL); VAN DER GREEF, Jan, NL-3971 BM Driebergen (NL)
(74) Representative: van Loon, C.J.J.
(86) International application number: PCT/NL2004/000616
(87) International publication number: WO 2005/024420

(56) References cited:
- WO-A-97/20076
- WO-A-98/57174
- WO-A-03/017177
- US-A1- 2003 044 846
- US-A1- 2003 096 309
- WANG ET AL PHYTOTHERAPY RESEARCH vol. 19, no. 3, March 2005, pages 173 - 182

## Description

The present invention is related to the field of life sciences and the domain of health and diseases, in particular the development of strategies and products for the prevention, treatment or curing of a disease.

In contrast with the reductionistic approach as conventionally applied by pharmaceutical companies to develop new medicaments, the present invention is based on a holistic approach to living organisms.

Such a holistic approach to living organisms has, for instance, been the basis of applying herbal medicine such as Traditional Chinese Medicine (TCM) from its earliest years. An important starting point in herbal medicine based approaches is that every healthy organism is in balance. Balance is considered to be a complex interplay between body and mind, which is reflected at all levels ranging from the biochemical component perspective to the energetic system control of our physical body. Internal imbalances can stem from a wide variety of factors and lead to a plethora of conditions ranging from short perturbations to chronic disease processes. Additionally, in such an approach as in TCM the uniqueness of each human being is recognized and drives the necessity to develop a personalized medication to obtain optimal results based on multi-component treatments.

At a first glance the "Western" medical approach may seem very different from this. However, the revolution in genomics that has taken place in life sciences during the last decade has provided considerable support for a more holistic view on diagnosis and treatment. Furthermore, the issue of personalized medicine is now receiving considerable attention due to the new insights in i.a. pharmacogenomics. Although the principle of homeostasis has been a cornerstone of Western physiology for more than a century, the enormous complexity of biological systems has often driven pharmaceutical research towards trying to identify and influence only single targets that make the difference between health and disease. This approach has indeed yielded many potent drugs but also revealed major drawbacks. In fact one tries to influence a system by interacting with a single protein that is often part of a complex pathway and involved in a cascade of reactions and feed-back loops. In this respect reference can, for instance, be made to US 2003/096309 A1 which relates to a target-centric method for identifying drug-drug interactions and which uses as basis pure components. Said method is limited to either synthesized or purified molecules from which only partial synergetic information can be obtained by random screening efforts. In said document a screening method is described for the identification of combinations of known drugs (in particular small organic molecules) that exhibit previously unknown effects even where each drug have previously exhibited little or none of these effects. To identify such combinations use is made of a conventional drug library and consequently a very large number of assay measurements at cellular levels. It does not teach or suggest a method wherein the impact of a multicomponent natural product mixture is determined on the profile of a disease within a group of living systems. The reality is that most diseases are multi-factorial which means that treating a single target provides a partial treatment (reduction of symptoms) and in the majority of cases no cure. Although this awareness is not new, it has been impossible to find alternative routes giving the mentioned complexity of the system.

A method has, however, now been developed that enables highly detailed profiling of complex mixtures and subsequent measurements of the complex multicomponent induced changes in *in-vitro* systems (such as cell cultures). In this method the interaction of multiple components in complex mixtures such as natural products or extracts and mixtures thereof (including nutraceutical products, functional food products, herbal medicinal products, other natural compounds and biofluids) with living biological systems can very effectively be measured, using a particular set of steps wherein technologies are applied such as biostatistics and bioinformatics. By means of such measurements the impact of multicomponent mixtures on the biological profile of a disease can advantageously be determined. Moreover, such measurements enable the identification of the effective components within the multicomponent mixtures and their respective concentrations required for having an impact on the biological profile of the disease can be identified.

Accordingly, the present invention relates to a method as defined in claim 1.

In a preferred embodiment of the present invention the multicomponent mixture is optimized towards the optimal activity for curing or preventing the development of a disease state of a living system both in view of efficacy and safety (cure or treat).

Hence, the present invention also relates to a method according to the present invention, wherein step (d) is followed by a step (e) in which a set of multicomponent natural product mixtures is prepared on the basis of the information obtained in step (d) which mixtures are expected to display the desired impact on the biological profile of the disease, whereby step (e) is followed by a step (f) wherein the impact on the biological profile of the disease is determined of the set of multicomponent mixtures as prepared in step (e), using multivariate analysis.

Accordingly, the present invention also relates to a method as defined in claim 2.

In another preferred embodiment of the present invention, in a step (g) one or more of the multicomponent mixtures as prepared in step (e) are selected which mixtures display in step (f) a desired and improved impact on the biological profile of the disease. The one or more multicomponent mixtures thus selected in step (g) display an improved impact on the biological profile of the disease when compared with the other multicomponent mixtures prepared in step (e).

In another attractive embodiment of the present invention, the one or more of the selected multicomponent mixtures as obtained in step (g) are adjusted by means of adding one or more further components to the mixture(s), whereafter the impact of the adjusted mixture on the biological profile of the disease is determined using multivariate analysis.

Another advantage of the present invention is the fact that multicomponent natural product mixtures can be developed within a defined quality range. This is of significant importance because the quality of the respective natural components or groups of natural components may differ locally due to for instance different weather conditions during culturing, despite the implementation of good agriculture practice. If one or more of the natural components or one or more groups of natural components would not be of the desired quality, the multicomponent mixture can be adjusted in respect of said one or more natural components or groups of natural components so as to have the desired impact on the biological profile of the disease. This can, for instance, be established by adjusting the amount of the respective one or more natural components or one or more groups of natural components so as to obtain the required concentration(s) of the one or more natural components or one or more groups of natural components, ensuring the multicomponent mixture will have the desired impact on the biological profile of the disease.

Hence, in a preferred embodiment of the present invention the concentrations of at least one natural component or group of natural components of the mixture is adjusted to ensure that the at least one natural component or group of natural components of the mixture have the desired impact on the biological profile of the disease.

In accordance with the present invention, the development and quality control of natural product-based medicines can very attractively be established.

In the context of the present invention, a complex multicomponent natural product mixture is defined as a mixture of natural components or groups of natural components, which components are not produced by chemical synthesis but by a natural process. A multicomponent herbal mixture will, for instance, comprise two or more herbs which herbs as such may consist of a number of different components.

The living systems include human beings and all sorts of animals. When use is made of animals, the group of living systems is suitably selected from one particular type of animal.

The method according to the present invention enables the measurement of the effects of multiple target interventions and the development of products to optimally perform such interventions by a unique approach, revealing the biological profile of the effective components. The present invention provides, therefore, an important bridge function between the two complementary approaches used in TCM and Western Medicine, because it can reveal besides the effects of simple perturbations like a single drug also the complex perturbations using multicomponent mixtures such as, for instance, herbal medicine products and functional food products. This unique approach is referred to as multidimensional pharmacology (MDP) and uses the Systems Biology approach in which biological systems are studied by measuring and integrating metabolic data and other profile data, such as genetic and/or proteomic data.

The determination of biological effects and in particular synergetic multicomponent effects in accordance with the present invention is illustrated by the example of herbal medicine products in intervention strategies. The determination of such effects is not limited to biological and synergetic effects in mammalian systems but can address all possible forms of living systems with complex mixtures derived from the same portfolio of life.

The present invention is not limited to a particular type of disease, but embraces any disease of which a biological profile can be determined and can equally be applied in preventive applications.

A considerable advantage of the present invention resides in the fact that it provides a scientific basis for both efficacy and safety of the tested multicomponent mixtures.

The method of the present invention allows characterization of the complex multicomponent mixtures, in the case of TCM-products, the effects of these products in mammalian systems to reveal biological effects and the effects of TCM on patients.

A further very important aspect of the method according to the present invention is that it allows the measurement of all biological effects including additive and synergetic effects.

In health and disease studies a bodyfluid profile of for instance a plasma sample from a control group (reference group) and patient group (a group with symptoms of the disease with the biological profile) can be used to measure as many components as possible and is evaluated for differences in single components or patterns of components between the two groups to obtain a better insight in the underlying biological mechanisms, to detect novel biomarkers/surrogate markers, to predict toxicology or pharmacological response or to develop novel intervention routes. In the context of the present application a biomarker is defined as a characteristic that is objectively measured and evaluated as an indicator of: normal biological processes, pathogenic processes or pharmacological response to a therapeutic intervention. Biomarkers can be genes, transcripts, proteins, metabolites, (trace)-elements or any combination of those components.

The concept of the present invention of using patterns for dynamic modeling directly or after non-linear or linear multidimensional compression opens the unique route of studying dynamical processes using systems descriptions based on component patterns. In such studies perturbations as for instance via drug intervention routes can be monitored and evaluated in a multidimensional way and methods such as time series analysis, time warping and non-linear dynamic techniques can be applied. In the evaluation of data generated with the method of the present invention also the addition of other data and information from other sources can be of importance such as information from clinical dossiers of patients describing the medical diagnosis and clinical chemistry, or disease studies data on behavior, cognition, psychological, social, etc., levels. These data can be included or linked to the data set created by the method according to the present invention to classify patients or to discover sub-classes or other relevant observations. Especially in the design of new herbal medicine animal models are often preferred to yield biological activity, for instance the measure of insulin resistance in the study of metabolic syndrome (obesity, diabetes II, hypertension and other CV related diseases). The direct correlation of such data with the composition of the herbal mixture and the biological response profile in that model is a preferred embodiment of this invention when new mixtures are studied. When mixtures have already been proven to be safe in man or animal in case of veterinary applications, biomarker profiles of man can be used directly for the evaluation.

The application of the present invention creates a wide variety of novel approaches in using metabolomics, proteomics and/or other component profiles of bodyfluids to enhance the overall process of biomedicine or drug discovery, development including clinical evaluation, diagnostic applications and post-marketing surveillance. The created profiles can be used first of all to obtain a better insight in the underlying biological process a.o. for toxicology evaluation (predictive toxicology), biomarker/surrogate marker or biomarker/surrogate marker pattern discovery, protein target validation, comparison of animal models and relating these to human studies, phenotyping typically at the metabolite and/or protein level, responder/non-responder evaluation, validation of animal models such as transgenic models, providing the ability to correlate metabolite level data with other levels in systems biology such as gene, mRNA, RNAi and protein data.

The method according to the present invention generates patterns of components and the used multivariate analysis generates patterns of relevant components for a given situation or investigation. In modern biology and related nutraceutical, pharmaceutical and biotechnological industries this is a crucial new paradigm for driving the scientific research and industrial discovery and development processes. The basic understanding is that biology in general and disease processes in particular are multifactorial of nature and therefore the understanding of such processes requires a description or understanding based on a multitude of components. In most cases development processes are for instance based on a single target evaluated with a single biomarker for efficacy or for differentiating of control groups from patients groups. The present invention, however, provides a method that allows the creation of a breakthrough in the ability to describe multifactorial diseases by multifactorial patterns as well as multifactorial responses toward multifactorial input variables such as in combination therapies (chemotherapy) or in evaluation of herbal medicine, functional food and nutraceutical responses.

In accordance with the present invention preferably at least one spectrometric technique, at least one electromigration-based technique or at least one chromatographic technique is used in step (a) to determine the profile of the disease. More preferably, use is made of two or more spectrometric techniques but special detection techniques for example laser-induced fluorescence and electrochemical detection in combination with separation techniques ((nano)-HPLC, electromigration-based methods) are included as well. Most preferably, use is made of at least a nuclear magnetic resonance technique and/or a mass spectrometry technique to determine the profile of the disease in step (a).

The biological profile to be determined in step (a) includes one or more metabolic, genetic and/or polemic profiles. Any combination of these profiles can be used. Preferably, such combination includes one or more metabolic profiles. More preferably, the biological profile includes metabolic, genetic and proteomic profiles.

In step (a) preferably the biological profile is determined of at least one type of bodyfluid or at least one type of tissue. More preferably, in step (a) the biological profiles are determined of at least two different types of bodyfluid.

The biological profiles are determined in step (a) using one or more of the following biomarkers; genes, transcripts, proteins, metabolites and (trace) elements.

In step (b) preferably use is made of at least one spectrometric technique, at least one electromigration-based technique or at least one chromatographic technique to determine the impact of the series of samples of the multicomponent mixture on the biological profile of the disease. More preferably, use is made of two or more spectrometric techniques, whereas most preferably use is made of at least a nuclear magnetic resonance technique and/or a mass spectrometry technique. In the case of protein profiling other techniques such as gel-based electrophoretic techniques are included as well.

In step (c) preferably use is made of at least one spectrometric technique, at least one electromigration-based technique or at least one chromatographic technique to determine the composition of the samples. More preferably, use is made of two or more spectrometric techniques, whereas most preferably use is made of at least a nuclear magnetic resonance technique and/or a mass spectrometry technique. In step (c) the number of samples of which the composition is determined in (c) is preferably at least 2, and more preferably in the range of 5-100.

In step (d) preferably use is made of at least one spectrometric technique, at least one electromigration-based technique or at least one chromatographic technique to identify the effective components or groups of components and their respective concentrations required for having an impact on the biological profile of the disease. More preferably, use is made of two or more spectrometric techniques. Most preferably use is made of at least a nuclear magnetic resonance technique and a mass spectrometry technique in step (d).

In step (f) preferably use is made of at least one spectrometric technique, at least one electromigration-based technique or at least one chromatographic technique to identify the effective components or groups of components and their respective concentrations required for having an impact on the biological profile of the disease. More preferably, use is made of two or more spectrometric techniques. Most preferably use is made of at least a nuclear magnetic resonance technique and a mass spectrometry technique in step (e).

The multicomponent mixture to be used in accordance with the present invention comprises a nutraceutical product, functional food product, herbal, algae, microbial, fungal medicinal product or (extract of) a biofluids. Preferably, the multicomponent natural product mixture is a herbal mixture.

In each of steps (a)-(d) and (f)(e) preferably use of at least one spectrometric technique. Suitably a nuclear magnetic resonance technique ("NMR") or mass spectrometry technique ("MS") can be used, whereby the latter technique focuses on a limited number of small molecule compounds. Both of these techniques have however limitations. The NMR approaches are limited in that they typically provide reliable information only of compounds present at high concentration. On the other hand, global or focused mass spectrometry techniques do not require high concentrations but can provide information at a broad screening level or of only limited portions of a biological profile. As used herein, the terms "small molecule" and "metabolite" are used interchangeably. Small molecules and metabolites include, but are not limited to, lipids, steroids, amino acids, organic acids, bile acids, eicosanoids, peptides, carbohydrates and trace elements.

Therefore, in each of steps (a)-(d) and (f)(e) preferably use is made of at least a nuclear magnetic resonance technique and/or a mass spectrometry technique with a preference for mass spectrometry in the field of protein profiling (proteomics).

The situation for small molecules is discussed as an example. Sample preparation for NMR can generally be very straightforward using freeze-drying and reconstitution in D₂O because the focus is on the higher concentration components, i.e. of typical concentration > 100 nanogram/mL. For MS a variety of sample preparation approaches can be used ranging from solid phase extraction and liquid/liquid extraction to more specific methods using, for instance, affinity-based methods or derivitization procedures both for GC-MS as well as LC-MS.

In each of steps (a)-(d) and (f)e) use can be made of spectrometric data obtained from one or more platforms including, but not limited to, MS, NMR, liquid chromatography ("LC"), gas-chromatography ("GC"), high performance liquid chromatography ("HPLC"), capillary electrophoresis ("CE"), and any known form of hyphenated mass spectrometry in low or high resolution mode, such as LC-MS, GC-MS, CE-MS, LC-UV, MS-MS, MSⁿ, etc. Typical profile data can also be obtained by using more component specific detectors such as laser-induced fluorescence and electrochemical detection.

As used herein, the term "spectrometric data" includes data from any spectrometric or chromatographic technique. Spectrometric techniques include, but are not limited to, resonance spectroscopy, mass spectroscopy, and optical spectroscopy. Chromatographic techniques include, but are not limited to, liquid phase chromatography, gas phase chromatography, and electrophoresis.

If a spectral profile is obtained, as with standard spectroscopic methods, a primary necessary step is to adjust for minor shifts in the spectra both in the intensity dimension as well as in the spectral or chromatographic dimension. Shifts can be due to instrumental factors, environmental conditions, or to varying concentrations of components (as is often the case in urine analysis). As an example, variation in NMR chemical shifts often occurs and needs to be accounted for, but the reproducibility and standardization in the intensity (or peak area) of a single profile (quantitation dimension) is typically very satisfactory. This is in contrast to MS where the peak intensity (ion abundance) dimension needs to be carefully adjusted or standardized due to lack of calibrants for each component present in the profile. In hyphenated techniques the reproducibility of the separation method (GC, LC or electromigration driven techniques such as capillary electrophoresis (CE)) needs to be carefully evaluated as well. In this respect near-infrared spectral profiles are impressive and correction in either dimension is hardly required.
In general, small instrumental shifts in the spectral (variable) dimension will be falsely interpreted as representing different components when a collection of data profiles is subjected to pattern recognition analysis. A straightforward way to cope with this problem is by using binning techniques in which the spectrum is reduced in resolution to a sufficient degree to insure that a given peak remains its bin despite small spectral shifts between analyses. For example, in NMR the chemical shift axis may be descretized and coarsely binned, and in MS the spectral accuracies may be rounded to integer atomic mass unit values. However, more subtle procedures are preferred such as partial linear fit for NMR or other alignment procedures for MS.

After initial data pre-processing the spectral profiles are set for pattern recognition (multivariate analysis).

The ability to use different techniques to produce bodyfluid profiles is optimally used by multiway multivariate analysis and allows the measurement of different bodyfluids of the same system (such as plasma and urine or plasma and CSF) to reveal novel insights into the systems biology, for instance the effect of the blood brain barrier when comparing plasma and urine profiles. Hence, in each of steps (a)-(d) preferably use is made of multiway multivariate analysis.

The present invention provides a method of spectrometric data processing utilizing multiple steps of multivariate analysis to process data in a hierarchal procedure (steps (a)-(d) and (f)e)). In each of steps (a)-(d) and (f)e) a first multivariate analysis can be used on a plurality of data sets to discern one or more sets of differences and/or similarities between them, whereafter a second multivariate analysis can be used to determine a correlation (and/or anti-correlation, i.e., negative correlation) between at least one of these sets of differences (or similarities) and one or more of the plurality of data sets. In step (a) the determination of the biological profile of a disease may also be based on the correlation.

As used herein, the term "data sets" refers to the spectrometric data associated with one or more spectrometric measurements. For example, where the spectrometric technique is NMR, a data set may comprise one or more NMR spectra. Where the spectrometric technique is UV spectroscopy, a data set may comprise one or more UV emission or absorption spectra. Similarly, where the spectrometric technique is MS, a data set may comprise one or more mass spectra. Where the spectrometric technique is a chromatographic-MS technique (e.g., LC-MS, GC-MS, etc), a data set may comprise one or more mass chromatograms. Alternatively, a data set of a chromatograms or reconstructed TIC chromatograms. In addition, it should be realized that the term "data sets" includes both raw spectrometric data and data that has been pre-processed (e.g., to remove noise, baseline, detect peaks, etc.).

Moreover, as used herein, the term "data sets" may refer to substantially all or a sub-set of the spectrometric data associated with one or more spectrometric measurements. For example, the data associated with the spectrometric measurements of different sample sources (samples of groups with symptoms of the disease (experimental group samples) v. samples of reference or healthy groups (control group samples)) may be grouped into different data sets. As a result, a first data set may refer to experimental group sample measurements and a second data set may refer to control group sample measurements. In addition, data sets may refer to data grouped based on any other classification considered relevant.

The present invention also provides a method of spectrometric data processing utilizing multivariate analysis to process data at two or more hierarchal levels of correlation. In each of steps (a)-(d) and (f)e) use can be made of a multivariate analysis on a plurality of data sets to discern correlations (and/or anti-correlations) between data sets at a first level of correlation, whereafter the multivariate analysis can be used to discern correlations (and/or anti-correlations) between data sets at a second level of correlation. In step (a) the determination of the biological profile of a biological system may also be based on the correlations discerned at one or more levels of correlation.

In accordance with the present invention the spectrometric data processing in each of steps (a)-(d) and (f)e) can be carried out utilizing multiple steps of multivariate analysis to process data sets in a hierarchal procedure, whereby one or more of the multivariate analysis steps further comprises processing data at two or more hierarchal levels of correlation. For example, in each of steps (a)-(d) a first multivariate analysis can be used on a plurality of data sets to discern one or more sets of differences and/or similarities between them; a second multivariate analysis can be used to determine a first level of correlation (and/or anti-correlation) between a first set of differences (or similarities) and one or more of the data sets; and the second multivariate analysis can be used to determine a second level of correlation (and/or anti-correlation) between the first set of differences (or similarities) and one or more of the data sets. In step (a) the determination of the biological profile of the disease can be based on the correlations discerned at one or more levels of correlation.

Suitable forms of multivariate analysis include, for example, principal component analysis ("PCA"), discriminant analysis ("DA"), PCA-DA, factor analysis, canonical correlation ("CC"), partial least squares ("PLS"), predictive linear discriminant analysis ("PLDA"), neural networks, multilevel/multiway/multiblock analysis, iterative target analysis, general procrustus analysis, support vector machines ("SVM"), parafac and pattern recognition techniques.

The use of the above-described multivariate analysis techniques is well-known in the art. For a more detailed description reference van, for instance, be made to pending US Provisional Patent Application, Serial No. 60/312,145 (Method and System for Profiling Biological Systems).
To extract the maximum value from the data, multivariate analysis tools as outlined above may be used in concert with additional statistical and informatics strategies. Once statistically significant differences in, for instance, metabolite abundances are determined and quantified among groups of samples, the objective becomes to understand the underlying biological reasons for and the contexts of the results. A first step is to identify metabolic components observed in data spectra and revealed by multivariate analysis to be significant differences among samples. Such identification typically involves querying various databases of known metabolite component spectra and structures. A next step is to mine existing knowledge about molecular interactions through searches of public and private databases. This can go some way in explaining the associations and behaviour observed in the metabolomic profile results. However, because most of the metabolic, genomic, proteomic, and interaction databases depict biochemical events in a static state, progressively sophisticated analytical and mathematical tools are needed to integrate disjointed biological clues into dynamic models that are better suited to explain, for example, pathological processes.

Indeed, both linear and non-linear multivariate analyses may uncover statistically significant associations between biomolecular components that will not be explained through the mining of existing databases or literature.

A preferred embodiment of the method of the present invention comprises the following steps :
1. A selection is made of the relevant samples, for instance bodyfluids (plasma, urine, CSF, saliva, synovial fluid etc.)
2. A selection is made of the width of the biological profiling; transcripts, proteins, metabolites, etc.
3. A sample is prepared based on the spectrometric techniques to be used for determining the biological profile (e.g. GCMS, LCMS, CEMS, MS/MS combinations various NMR-methodologies, gel-based electrophoretic techniques etc.)
4. The profile is determined using the spectrometric techniques, gel-based techniques, NMR-profiles and preferred MS-approaches in case of metabolomics covering lipids, steroids, bile acids, eicosanoids, (neuro)peptides, vitamins, organic acids, neurotransmitters, amino acids, carbohydrates, ionic organics, nucleosides, inorganics, xenobiotics, etc., with a preference to include peptides. Also a global MS-profile to describe in a single experiment the major high concentration components can be included which is often a good indication for the balance/homeostasis of a system in addition to the NMR-profile.
5. The data obtained is preprocessed using preferable the technique described in Dutch patent application 1016034, in combination with PCA-DA, multiblock/multiway multivariate analysis based on linear and non-linear techniques and the partial linear fit algorithm.
6. The outcome of 4 is combined with other relevant data sources such as medical history, clinical chemistry records, medical description and behavioural, social, psychological data, etc.
7. The (non-linear or linear) dynamics of dynamical diseases are studied by using one of the profiling-components or any combination of the profiling components, preferably by using a combination of non-linear compression and dynamic modeling techniques.

The concept of the present invention is suitably based on the following aspects, the profiling of complex mixtures such as body fluids by a combination of NMR and a selection of hyphenated mass spectrometric techniques (GC-,LC-,-CE-MS/MS, ICPMS), the evaluation of the combination with data preprocessing/scaling preceding multiblock/multiway multivariate analysis, the combination of instrumental datasets created with other relevant datasets, the linking of data sets arising from samples from one system but via different bodyfluid profiles and the ability to study all forms of non-linear dynamics.

In cases where biological active components or groups of active components are detected and the composition constraints for a desired effect are revealed, the knowledge can be used to design mixtures or novel herbal medicine.

The detection of biological active patterns in mixtures such as herbal products enables also the focused quality control or optimized production of plants enabling efficient and well-controlled products. This is a crucial step and a major bottleneck in the current status of quality control of natural products. Although spectroscopic techniques have been suggested for controlling the composition of a mixture, see for instance W00047922 (Dunn et al.), the inability to link a concentration change of a component or a chance in composition ratio between multiple components in a product with biological activity prohibits the mandatory need for concentration-effect control. The present invention constitutes therefore a breakthrough as both the profiling of the multicomponent product and the profiling of the biological response can be performed, and both profiles so obtained can be correlated. Therefore, the present disclosure also relates to a method for designing a multicomponent mixture and controlling the composition of a multicomponent mixture, wherein the concentration of at least one natural component or at least one group of natural components of the mixture is adjusted to ensure that the at least one natural component or group of natural components of the mixture has an impact on a biological profile of the disease. The multicomponent mixture can for instance be contained in a plant which quality is controlled and adjusted to produce a high quality product.

The detection of biological active patterns in mixtures such as herbal products enables also the focused quality control or optimized production of plants enabling efficient and well-controlled products.

In cases where all or part of the biological active components are detected and the composition constraints for optimal effect are revealed, the knowledge can be used to design mixtures of synthetically obtained components. In the latter case pharmaceutical preparations can be generated.

The present invention also relates to the use of the present method for setting up breeding programs, Good Agriculture/Manufacture Practice (GAP/GMP) protocols and post-harvesting processing of natural products for use in natural product -based medicines.

### Example

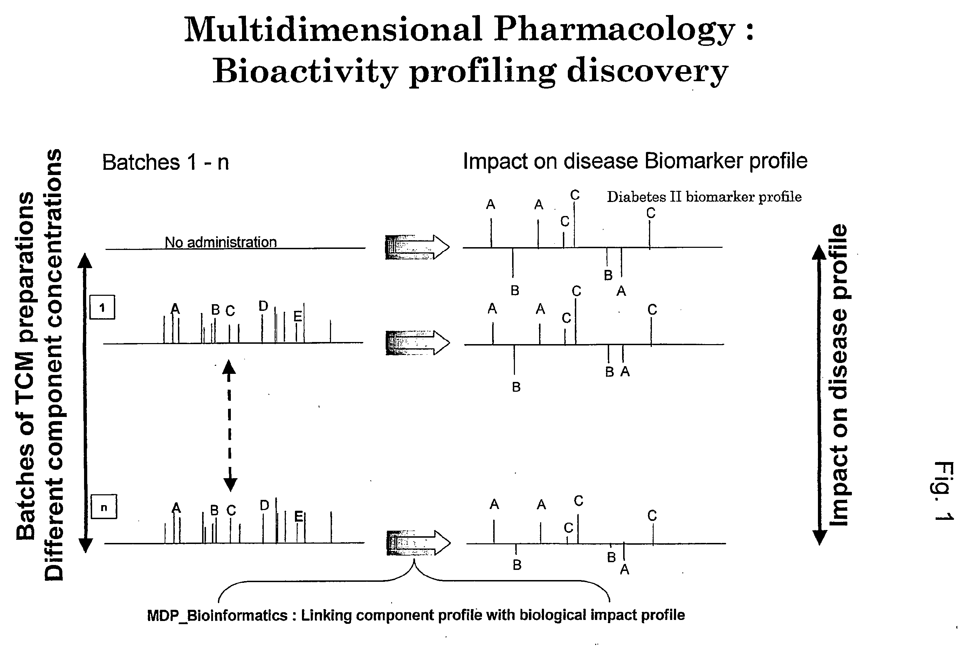

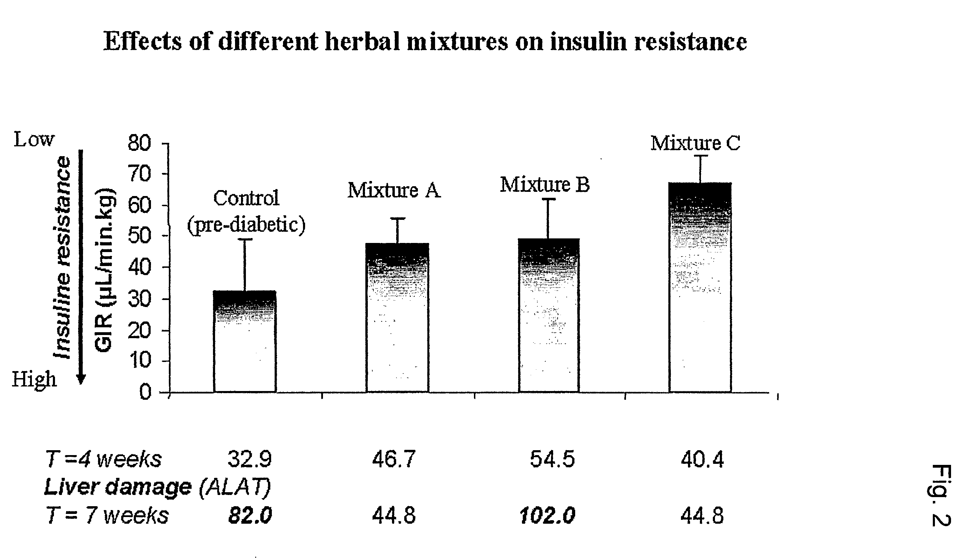

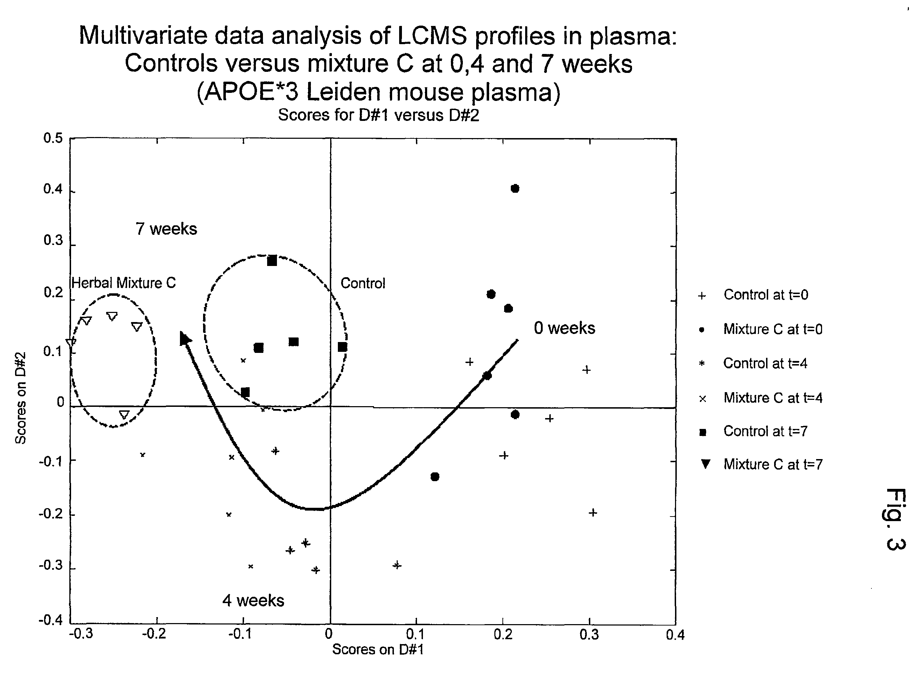

The method according to the present invention is schematically depicted in Figure 1. A typical experiment for herbal medicine in accordance with the present invention based on the fingerprints as generated by the systems biology approach a typical experiment is based on the following steps:
1. A number of batches of a mixture or a set of different herbal mixtures, preferably with a significant variation in composition, is measured (fingerprinted) by a profiling technique such as NMR or mass spectrometry; indicated as batches 1-n in the left part of Figure 1.
2. The profiles of the effect of those batches after administration into an animal model or in a human trial are measured as well as a reference group which is not treated (comprised of both disease animals and wildtype or healthy subjects/patients) or chosen to be treated in another way.
3. The reference group provides the biomarker profile for the disease and the other experiments provide the impact of the mixture on the disease pattern and reveals also other effects. In addition clinical endpoints in human trials or typical biological effects measured in cell-models and/or animal models can be used for evaluation of the multicomponent mixture. This yields a prove of effect on the specific disease group. In case a control group is not available comparative analysis can be used to reveal the optimal biological effect towards a clinical endpoint or hypothesis.
4. (Non)-linear multivariate correlation of the patterns of the mixture components and the effect profiles including also all other information such as clinical endpoints or any biological effect in other models (cell based or animal models) enables the detection of the patterns of components responsible for the biological effects.
5. In designing herbal medicine, the composition of the product can be varied and evaluation based on a biological response in an animal model or disease biomarker fingerprint can be used to optimize for efficacy and safety. In Figure 2 an example is given of a readout of an animal experiment, measuring the effect of herbal mixtures on insulin resistance and liver safety. Different compositions yield different efficacy (Insuline resistance) and also display different safety profiles as measured via a liver enzyme (ALAT) in the model. In addition the biomarker fingerprint of each mixture can be evaluated as shown in Figure 3, in which the multivariate analysis of one of the mixtures is given compared to the control (disease) group as a function of time. Clearly the effect shows after 4 weeks of treatment and further analysis of the data yields component specific and biological pathway or system communication specific information. Correlation between the outcome of the experiments related to Figure 2 and 3 provide the information which component(s) or groups of components are responsible for the biological effect. Optimization can be performed with or without identification of the components of the mixture.
6. After designing the optimal multicomponent mixture the fingerprint of the mixture can be controlled based on the information as obtained under 4. Profiling of multicomponent mixtures such as herbal medicine can be performed with separation methods but more preferable with spectroscopic techniques such as NMR or mass spectrometric techniques using direct infusion with or GC/MS and LC/MS methodologies. Direct fingerprinting strategies based Fourier Transform Mass Spectrometry are especially attractive given the high resolution capabilities and unique identification power, which is for the complexity of herbal medicine products often preferred.
7. In case of growing and producing of plants the described invention can be used for optimal selection of the conditions of production/culturing of such plants and the quality control of the production.

## Claims

1. An *in vitro* method for identifying within a complex multicomponent natural product mixture the effective natural components or groups of natural components and their respective concentrations required for having an effect on the biological profile of a disease within a group of living systems, which mixture comprises a nutraceutical product, functional food product, herbal medicinal product, biofluid, an extract of a biofluid, or a mixture thereof, which method comprises the steps of:
(a) determining a biological profile of the disease by comparing the biological profile of a group of living systems with symptoms of the disease with the biological profile of a reference (or healthy) group of living systems, using a multivariate analysis, by measuring and integrating data from one or more metabolic, genetic and/or proteomic profiles;
(b) determining the impact of a series of samples of the multicomponent mixture on the biological profile of the disease, in which samples the concentrations of one or more natural components or groups of natural components differ, using a multivariate analysis;
(c) determining the composition of the samples of the multicomponent mixture that have shown in step (b) a desired impact on the biological profile of the disease, using a multivariate analysis;
(d) identifying within the compositions as determined in step (c) the effective natural components or groups of natural components and their respective concentrations required for having the desired impact on the biological profile of the disease, using a multivariate analysis.

2. Method according to claim 1, wherein step (d) is followed by a step (e) in which a set of multicomponent natural product mixtures is prepared on the basis of the information obtained in step (d), which mixtures are expected to display the desired impact on the biological profile of the disease, whereby step (e) is followed by a step (f) wherein the impact on the biological profile of the disease is determined of the set of multicomponent mixtures as prepared in step (e), using multivariate analysis.

3. Method according to claim 2, wherein from the set of multicomponent mixtures as prepared in step (e) one or more multicomponent mixtures are selected in a step (g), which selected multicomponent mixtures display a desired and improved impact on the biological profile of the disease.

4. Method according to any one of claims 1-3, wherein in step (a) use is made of at least one spectrometric technique, at least one electromigration-based technique and/or at least one chromatographic technique to determine the profile of the disease.

5. Method according to any one of claims 1-4, wherein in step (b) use is made of at least one spectrometric technique, at least one electromigration-based technique and/or at least one chromatographic technique to determine the impact of the series of samples of the multicomponent mixture on the biological profile of the disease samples.

6. Method according to any one of claims 1-5, wherein in step (c) use is made of at least one spectrometric technique, at least one electromigration-based technique and/or at least one chromatographic technique to determine the composition of the samples.

7. Method according to any one of claims 1-6, wherein in step (d) use is made of at least one spectrometric technique, at least one electromigration-based technique and/or at least one chromatographic technique to identify the effective components and their respective concentrations required for having an impact on the biological profile of the disease.

8. Method according to any one of claims 2-7, wherein in step (f) use is made of at least one spectrometric technique, at least one electromigration-based technique or at least one chromatographic technique to identify the effective components and their respective concentrations required for having an impact on the biological profile of the disease.

9. Method according to any one of claims 2-8, wherein use is made of two or more spectrometric techniques.

10. Method according to claim 9, wherein use is made of at least a nuclear magnetic resonance technique and a mass spectrometry technique.

11. Method according to claim 10, wherein the biological profile includes the metabolic, genetic and proteomic profiles.

12. Method according to any one of claims 1-11, wherein in (a) the biological profiles are determined of at least one type of bodyfluid.

13. Method according to any one of claims 1-12, wherein in (a) the biological profiles are determined of at least one type of tissue.

14. Method according to claim 12, wherein in (a) the biological profiles are determined of at least two different types of bodyfluid.

15. Method according to any one of claims 1-14, wherein in (a) the biological profiles are determined using one or more of the following biomarkers; genes, transcripts, proteins, metabolites and (trace) elements.

16. Method according to any one of claims 1-15, wherein the number of samples of which the composition is determined in (c) is at least 2.

17. Method according to claim 16, wherein the number of samples of which the composition is determined in (c) ranges from 5-100

18. Method according to any one of claims 1-17, wherein the multicomponent natural product mixture is a herbal mixture.

19. Method according to any one of claims 1-18, wherein the concentration of at least one natural component or group of natural components of the mixture is adjusted to ensure that the at least one natural component or group of natural components of the mixture has the desired impact on the biological profile of the disease.

20. Use of the method according to any one of claims 1-19, for setting up breeding programs, Good Agriculture/Manufacture Practice (GAP/GMP) protocols and post-harvesting processing of natural products for use in natural product -based medicines.

## Patentansprüche

1. *In vitro* Verfahren zum Identifizieren der wirksamen natürlichen Komponenten oder Gruppen von natürlichen Komponenten und ihrer entsprechenden Konzentrationen in einem komplexen Mehrkomponenten-Naturproduktgemisch, die erforderlich sind, um eine Auswirkung auf das biologische Profil einer Krankheit innerhalb einer Gruppe lebender Systeme zu haben, wobei das Gemisch ein Nutrazeutikum, ein funktionelles Lebensmittelprodukt, ein pflanzliches Medizinprodukt, ein Biofluid, einen Extrakt eines Biofluids, oder eine Mischung davon umfaßt, wobei das verfahren Schritte umfaßt, bei denen man:
a) ein biologisches Profil der Krankheit durch Vergleichen des biologischen Profils einer Gruppe lebender Systeme, mit Symptomen der Krankheit, mit dem biologischen Profil einer Referenz- (oder gesunden) Gruppe lebender Systeme, unter Verwendung einer multivariaten Analyse bestimmt, bei der Daten aus einem oder mehreren metabolischen, genetischen und/oder proteomischen Profil(en) gemessen und integriert werden;
b) die Auswirkung einer Serie von Proben des Mehrkomponenten-Gemischs auf das biologische Profil der Krankheit unter Verwendung einer multivariaten Analyse bestimmt, wobei sich in den Proben die Konzentrationen von einer oder mehreren natürlichen Komponente(n) oder Gruppe(n) von natürlichen Komponenten unterscheiden;
c) die Zusammensetzung der Proben des Mehrkomponenten-Gemischs, die in Schritt (b) eine gewünschte Auswirkung auf das biologische Profil der Krankheit gezeigt haben, unter Verwendung einer multivariaten Analyse bestimmt;
d) innerhalb der Zusammensetzungen, die in Schritt (c) bestimmt wurden, die wirksamen natürlichen Komponenten oder Gruppen von natürlichen Komponenten und ihre entsprechenden Konzentrationen, die erforderlich sind, um die gewünschte Auswirkung auf das biologische Profil der Krankheit zu haben, unter Verwendung einer multivariaten Analyse identifiziert.

2. Verfahren gemäß Anspruch 1, bei dem sich an Schritt (d) ein Schritt (e) anschließt, bei dem ein Satz von Mehrkomponenten-Naturproduktgemischen auf Basis der in Schritt (d) erhaltenen Information hergestellt wird, wobei von den Gemischen erwartet wird, daß sie die gewünschte Auswirkung auf das biologische Profil der Krankheit zeigen, wobei sich an Schritt (e) ein Schritt (f) anschließt, bei dem für den in Schritt (e) hergestellten Satz der Mehrkomponenten-Gemische die Auswirkung auf das biologische Profil der Krankheit unter Verwendung einer multivariaten Analyse bestimmt wird.

3. Verfahren gemäß Anspruch 2, bei dem aus dem Satz der Mehrkomponenten-Gemische, der in Schritt (e) hergestellt wurde, ein oder mehrere Mehrkomponenten-Gemisch(e) in einem Schritt (g) ausgewählt wird/werden, wobei die ausgewählten Mehrkomponenten-Gemische eine gewünschte und verbesserte Auswirkung auf das biologische Profil der Krankheit zeigen.

4. Verfahren gemäß einem der Ansprüche 1-3, bei dem in Schritt (a) von mindestens einer spektrometrischen Technik, mindestens einer elektromigrations-basierten Technik und/oder mindestens einer chromatografischen Technik Gebrauch gemacht wird, um das Profil der Krankheit zu bestimmen.

5. Verfahren gemäß einem der Ansprüche 1-4, bei dem in Schritt (b) von mindestens einer spektrometrischen Technik, mindestens einer elektromigrations-basierten Technik und/oder mindestens einer chromatografischen Technik Gebrauch gemacht wird, um die Auswirkung der Serie von Proben des Mehrkomponenten-Gemischs auf das biologische Profil der Krankheit/Proben zu bestimmen.

6. Verfahren gemäß einem der Ansprüche 1-5, bei dem in Schritt (c) von mindestens einer spektrometrischen Technik, mindestens einer elektromigrations-basierten Technik und/oder mindestens einer chromatografischen Technik Gebrauch gemacht wird, um die Zusammensetzung der Proben zu bestimmen.

7. Verfahren gemäß einem der Ansprüche 1-6, bei dem in Schritt (d) von mindestens einer spektrometrischen Technik, mindestens einer elektromigrations-basierten Technik und/oder mindestens einer chromatografischen Technik Gebrauch gemacht wird, um die wirksamen Komponenten und ihre entsprechenden Konzentrationen, die erforderlich sind, um eine Auswirkung auf das biologische Profil der Krankheit zu haben, zu identifizieren.

8. Verfahren gemäß einem der Ansprüche 2-7, bei dem in Schritt (f) von mindestens einer spektrometrischen Technik, mindestens einer elektromigrations-basierten Technik oder mindestens einer chromatografischen Technik Gebrauch gemacht wird, um die wirksamen Komponenten und ihre entsprechenden Konzentrationen, die erforderlich sind, um eine Auswirkung auf das biologische Profil der Krankheit zu haben, zu identifizieren.

9. Verfahren gemäß einem der Ansprüche 2-8, bei dem von 2 oder mehr spektrometrischen Techniken Gebrauch gemacht wird.

10. Verfahren gemäß Anspruch 9, bei dem von mindestens einer kernmagnetischen Resonanztechnik und einer Massenspektrometrischen Technik Gebrauch gemacht wird.

11. Verfahren gemäß Anspruch 10, bei dem das biologische Profil die metabolischen, genetischen und proteomischen Profile umfaßt.

12. Verfahren gemäß einem der Ansprüche 1-11, bei dem in (a) die biologischen Profile von mindestens einer Art von Körperflüssigkeit bestimmt werden.

13. Verfahren gemäß einem der Ansprüche 1-12, bei dem in (a) die biologischen Profile von mindestens einer Art von Gewebe bestimmt werden.

14. Verfahren gemäß Anspruch 12, bei dem in (a) die biologischen Profile von mindestens zwei verschiedenen Arten von Körperflüssigkeit bestimmt werden.

15. Verfahren gemäß einem der Ansprüche 1-14, bei dem in (a) die biologischen Profile unter Verwendung eines oder mehrerer der folgenden Biomarker bestimmt werden; Gene, Transkripte, Proteine, Metabolite und (Spuren-) Elemente.

16. Verfahren gemäß einem der Ansprüche 1-15, bei dem die Anzahl der Proben, von denen die Zusammensetzung in (c) bestimmt wird, mindestens zwei ist.

17. Verfahren gemäß Anspruch 16, bei dem die Anzahl der Proben, von denen die Zusammensetzung in (c) bestimmt wird, von 5-100 reicht.

18. Verfahren gemäß einem der Ansprüche 1-17, bei dem das Mehrkomponenten-Naturproduktgemisch ein pflanzliches Gemisch ist.

19. Verfahren gemäß einem der Ansprüche 1-18, bei dem die Konzentration von mindestens einer natürlichen Komponente oder Gruppe von natürlichen Komponenten des Gemischs angepaßt wird, um sicherzustellen, daß die mindestens eine natürliche Komponente oder Gruppe von natürlichen Komponenten des Gemischs die gewünschte Auswirkung auf das biologische Profil der Krankheit hat.

20. Verwendung des Verfahrens gemäß einem der Ansprüche 1-19, zum Erstellen von Zuchtprogrammen, "Guten Landwirtschafts/Hersteller-Praxis" (GAP/GMP) Protokollen und post-Ernte-Verarbeitungen von Naturprodukten für die Verwendung in Naturprodukt-basierten Arzneien.

## Revendications

1. Procédé *in vitro* pour identifier, à l'intérieur d'un mélange de produits naturels multicomposant complexe, les composants naturels efficaces ou groupes de composants naturels et leurs concentrations respectives nécessaires pour avoir un effet sur le profil biologique d'une maladie à l'intérieur d'un groupe de systèmes vivants, lequel mélange comporte un produit nutraceutique, un produit alimentaire fonctionnel, un produit de phytothérapie, un biofluide, un extrait d'un biofluide, ou un mélange de ceux-ci, lequel procédé comporte les étapes consistant à :
(a) déterminer un profil biologique de la maladie en comparant le profil biologique d'un groupe de systèmes vivants ayant des symptômes de la maladie au profil biologique d'un groupe de référence (ou sain) de systèmes vivants, en utilisant une analyse multivariée, en mesurant et en intégrant des données provenant d'un ou plusieurs profils métabolique, génétique et/ou protéomique,
(b) déterminer l'impact d'une série d'échantillons du mélange multicomposant sur le profil biologique de la maladie, dans laquelle des échantillons ont des concentrations d'un ou plusieurs composants naturels ou groupes de composants naturels diffèrent, en utilisant une analyse multivariée,
(c) déterminer la composition des échantillons du mélange multicomposant qui ont montré à l'étape (b) un impact voulu sur le profil biologique de la maladie, en utilisant une analyse multivariée,
(d) identifier dans les compositions comme déterminé à l'étape (c) les composants naturels efficaces ou groupes de composants naturels et leurs concentrations respectives nécessaires pour avoir l'impact voulu sur le profil biologique de la maladie, en utilisant une analyse multivariée.

2. Procédé selon la revendication 1, dans lequel l'étape (d) est suivie d'une étape (e) au cours de laquelle un ensemble de mélanges de produits naturels multicomposants est préparé sur la base des informations obtenues à l'étape (d), lesquels mélanges sont supposés afficher l'impact voulu sur le profil biologique de la maladie, de sorte que l'étape (e) est suivie d'une étape (f) au cours de laquelle l'impact sur le profil biologique de la maladie est déterminé par l'ensemble de mélanges multicomposants comme préparé à l'étape (e), en utilisant une analyse multivariée.

3. Procédé selon la revendication 2, dans lequel, à partir de l'ensemble de mélanges multicomposants comme préparé à l'étape (e), un ou plusieurs mélanges multicomposants sont sélectionnés lors d'une étape (g), lesquels mélanges multicomposants sélectionnés affichent un impact voulu et amélioré sur le profil biologique de la maladie.

4. Procédé selon l'une quelconque des revendications 1 à 3, dans lequel, à l'étape (a), on utilise au moins une technique spectrométrique, au moins une technique basée sur une électromigration et/ou une technique chromatographique pour déterminer le profil de la maladie.

5. Procédé selon l'une quelconque des revendications 1 à 4, dans lequel, à l'étape (b), on utilise au moins une technique spectrométrique, au moins une technique basée sur une électromigration et/ou une technique chromatographique pour déterminer l'impact de la série d'échantillons du mélange multicomposant sur le profil biologique des échantillons malades.

6. Procédé selon l'une quelconque des revendications 1 à 5, dans lequel, à l'étape (c), on utilise au moins une technique spectrométrique, au moins une technique basée sur une électromigration et/ou au moins une technique chromatographique pour déterminer la composition des échantillons.

7. Procédé selon l'une quelconque des revendications 1 à 6, dans lequel, à l'étape (d), on utilise au moins une technique spectrométrique, au moins une technique basée sur une électromigration et/ou au moins une technique chromatographique pour identifier les composants efficaces et leurs concentrations respectives nécessaires pour avoir un impact sur le profil biologique de la maladie.

8. Procédé selon l'une quelconque des revendications 2 à 7, dans lequel, à l'étape (f), on utilise au moins une technique spectrométrique, au moins une technique basée sur une électromigration ou au moins une technique chromatographique pour identifier les composants efficaces et leurs concentrations respectives nécessaires pour avoir un impact sur le profil biologique de la maladie.

9. Procédé selon l'une quelconque des revendications 2 à 8, dans lequel on utilise deux ou plus de deux techniques spectrométriques.

10. Procédé selon la revendication 9, dans lequel on utilise au moins une technique de résonance magnétique nucléaire et une technique de spectrométrie de masse.

11. Procédé selon la revendication 10, dans lequel le profil biologique inclut les profils métabolique, génétique et protéomique.

12. Procédé selon l'une quelconque des revendications 1 à 11, dans lequel, à l'étape (a), les profils biologiques sont déterminés à partir d'au moins un type de fluide corporel.

13. Procédé selon l'une quelconque des revendications 1 à 12, dans lequel, à l'étape (a), les profils biologiques sont déterminés à partir d'au moins un type de tissu.

14. Procédé selon la revendication 12, dans lequel, à l'étape (a), les profils biologiques sont déterminés à partir d'au moins deux types différents de fluide corporel.

15. Procédé selon l'une quelconque des revendications 1 à 14, dans lequel, à l'étape (a), les profils biologiques sont déterminés en utilisant un ou plusieurs des biomarqueurs suivants : gènes, produits de transcription, protéines, métabolites et éléments (trace).

16. Procédé selon l'une quelconque des revendications 1 à 15, dans lequel le nombre d'échantillons déterminé comme constituant la composition à l'étape (c) est au moins de 2.

17. Procédé selon la revendication 16, dans lequel le nombre d'échantillons déterminé comme constituant la composition à l'étape (c) se trouve dans la plage de 5 à 100.

18. Procédé selon l'une quelconque des revendications 1 à 17, dans lequel le mélange de produits naturels multicomposant est un mélange d'herbes.

19. Procédé selon l'une quelconque des revendications 1 à 18, dans lequel la concentration d'au moins un composant naturel ou groupe de composants naturels du mélange est ajustée pour garantir que le au moins un composant naturel ou groupe de composants naturels du mélange a l'impact voulu sur le profil biologique de la maladie.

20. Utilisation du procédé selon l'une quelconque des revendications 1 à 19, pour établir des programmes d'alimentation animale, protocoles Good Agriculture/Manufacture Practice (GAP/GMP) et un traitement post-récole de produits naturels destinés à être utilisés dans des médicaments à base de produits naturels.
